Europäisches Patentamt

European Patent Office

Office européen des brevets

(19)

(11) **EP 1 105 178 B1**

(12) **EUROPÄISCHE PATENTSCHRIFT**

(45) Veröffentlichungstag und Bekanntmachung des Hinweises auf die Patenterteilung:
**04.08.2004 Patentblatt 2004/32**

(21) Anmeldenummer: **99944442.5**

(22) Anmeldetag: **19.08.1999**

(51) Int Cl.⁷: **A61M 16/00**

(86) Internationale Anmeldenummer:
**PCT/EP1999/006080**

(87) Internationale Veröffentlichungsnummer:
**WO 2000/010633 (02.03.2000 Gazette 2000/09)**

(54) **VORRICHTUNG FÜR DAS SCHALTEN IN DIE EIN- ODER AUSATMUNGSPHASE BEI DER CPAP-THERAPIE**

DEVICE FOR SWITCHING THE INSPIRATION OR EXPIRATION PHASE DURING CPAP THERAPY

DISPOSITIF PERMETTANT DE PASSER EN PHASE INSPIRATOIRE OU EXPIRATOIRE DURANT UN TRAITEMENT PAR RESPIRATION SPONTANEE EN PRESSION POSITIVE CONTINUE

(84) Benannte Vertragsstaaten:
**AT BE CH CY DE DK ES FI FR GB GR IE IT LI LU MC NL PT SE**

(30) Priorität: **19.08.1998 DE 19837656**
**08.10.1998 DE 19846462**

(43) Veröffentlichungstag der Anmeldung:
**13.06.2001 Patentblatt 2001/24**

(73) Patentinhaber: **MAP Medizin-Technologie GmbH**
**82152 Martinsried (DE)**

(72) Erfinder:
• **MADAUS, Stefan**
**D-82152 Krailing (DE)**
• **VÖGELE, Harald**
**D-81245 München (DE)**
• **GRIEBEL, Jutta**
**D-86922 Pflaumdorf (DE)**

(74) Vertreter: **VOSSIUS & PARTNER**
**Siebertstrasse 4**
**81675 München (DE)**

(56) Entgegenhaltungen:
EP-A- 0 606 687      EP-A- 0 656 216
EP-A- 0 714 670      EP-A- 0 722 747
WO-A-93/08857        WO-A-98/35715

**Beschreibung**

Die Erfindung betrifft eine Vorrichtung für das Umschalten aus der Einatmungsphase in die Ausatmungsphase oder umgekehrt in der CPAP (continous positive airways pressure)-Therapie, bei der fortwährend ein positiver Luftdruck auf die Atmungswege (CPAP-Therapie) ausgeübt wird.

Die CPAP-Therapie dient der pneumatischen Schienung des Kehlkopfes durch kontinuierliche Zufuhr eines positiven Luftdrucks auf die Atemwege. Das Druckniveau wird dabei individuell auf den Patienten abgestimmt. Die Erfindung kommt zum Einsatz bei einem variierten CPAP-Verfahren, wobei das Ausatmen bei einem geringerem Druckniveau als das Einatmen erfolgt. Das hat den Vorteil, daß nicht gegen das hohe Druckniveau ausgeatmet werden muß. Für die Sicherheit des Patienten ist es von großer Bedeutung, daß die Einstellung der unterschiedlichen Druckniveaus beim Übergang von der Ein- zur Ausatmungsphase und umgekehrt mit hoher Genauigkeit erfolgt.

Üblicherweise wird im Stand der Technik der Atemgasfluß vom und zum Patienten gemessen und nach der Zeit differenziert (1. Ableitung), um einen deutlicheren Übergang zwischen der Ein- und der Ausatmungsphase zu erhalten. Die Ableitungen der Flanken der Gasflußkurve werden mit Schwellenwerten verglichen, die einen Übergang in die entsprechende andere Atmungsphase anzeigen. Der Atemgasfluß des Patienten kann jedoch Schwankungen enthalten; ferner können sich auch Einflüsse des Pulsschlages des Patienten im Gasfluß auswirken. Die Folge kann ein Überschwingen der ersten Ableitung sein, wobei der Schwellenwert für die Umschaltung in die andere Atmungsphase vor der erforderlichen Zeit erreicht wird, das andere Druckniveau eingeschaltet wird und die Sicherheit des Patienten gefährdet werden kann. Es ist aber auch möglich, daß der Patient zu flach atmet, so daß eine sehr geringe Steigung der Atemgasflußkurve in der Einatmungsphase auftritt. Die Folge ist ein so geringer Wert der 1. Ableitung, daß der Schwellenwert für den Übergang in die Einatmungsphase nicht erreicht wird.

Aus der EP-A2-0 656 216 ist ein Verfahren für das Schalten in die Einatmungs- oder Ausatmungsphase in der CPAP-Therapie bekannt. Dabei wird ein Generator zur Luftzufuhr für den Patienten für die Bestimmung der Ein- und Ausatmungsphase hinsichtlich der Motorgeschwindigkeit und des Stromverbrauchs überwacht und ein Operationssignal abgeleitet. Aus dem Operationssignal wird ein Signal, das für den Gasfluß zum Patienten repräsentativ ist, abgeleitet. Das nach der Zeit abgeleitete Flußsignal d"flow"/dt wird mit einem ersten und einem zweiten Schwellenwert verglichen und daraus die Änderung der Atmungsphase abgeleitet.

Aus EP-A-0 722 747, EP-A-0 714 670, WO-A-93/08857 sind weitere Verfahren und Vorrichtungen bekannt geworden.

Der Erfindung liegt die Aufgabe zugrunde, eine verbesserte Vorrichtung für das Schalten in die Ein- oder Ausatmungsphase in der CPAP-Therapie zur Verfügung zu stellen, wobei der Übergang in die Ein- oder Ausatmungsphase mit hoher Genauigkeit erkannt und die Sicherheit des Patienten erhöht wird.

Die Aufgabe wird mit den Merkmalen der Patentansprüche gelöst.

Bei der Lösung geht die Erfindung von folgenden Grundgedanken aus.

Die erste Ableitung einer Atemgasflußkurve von und zum Patienten wird mit zwei aufeinanderfolgenden Schwellenwerten für die Variation der ersten Ableitung verglichen. Dabei ist der erste Schwellenwert größer und daher unempfindlicher. Schwankungen, wie sie oft zu Beginn einer Atemphase auftreten, können diesen hohen Schwellenwert nicht erreichen und somit keine irrtümliche Schaltung eines Druckniveaus der CPAP-Therapie auslösen. Anschließend an den hohen Schwellenwert wird ein niedriger Schwellenwert, der daher empfindlicher ist, festgesetzt, um den Übergang in die nächste Atmungsphase mit hoher Genauigkeit festzustellen. Die Schaltung in die andere Atmungsphase erfolgt, wenn die erste Ableitung den zweiten Schwellenwert erreicht hat.
Bei forcierter Atmung kann auch die hohe Schwelle überschritten werden, daher ist die spontane Atmung jederzeit möglich.

Falls der vorstehend erläuterte Schaltvorgang in die Ausatmungsphase nicht ausgelöst wird, erfolgt die Umschaltung aufgrund einer begrenzenden Zeitschaltung, z.B. spätestens nach 3 bis 4 s für die Einatmungsphase.

Bei einer besonderen Ausführungsform wird zusätzlich zum Vergleich mit dem zweiten niedrigen Stellenwert in der Ausatmungsphase der zeitliche Verlauf des Gasflusses in mindestens einer Folge von drei Abtastwerten abgetastet.

Wenn bei einer derartigen Folge die Bedingung erfüllt ist, daß der zweite und der dritte Abtastwert den jeweils vorangegangenen Abtastwert um einen vorgegebenen Betrag $\Delta$ übersteigt, also eine monotone Steigung des Atemgasflusses vorliegt, so erfolgt das Schalten in die Einatmungsphase bei dem dritten Abtastwert; falls diese Umschaltung nicht erfolgt, wird die Schaltung in die Einatmungsphase durch Vergleichen der ersten Ableitung mit dem niedrigen Schwellenwert ausgelöst.

In einer bevorzugten Ausführungsform wird sowohl die 1. Ableitung des Atemgasflusses als auch der Atemgasfluß selbst mit zugehörigen Schwellenwerten überwacht und in die Einatmungsphase umgeschaltet, wenn einer der beiden Parameter den zugehörigen Schwellenwert erreicht.

Eine weitere besondere Ausführungsform betrifft ein Verfahren zur Erkennung und Ausschließung von Signalen in der Atemgasflußkurve, die vom Pulsschlag (Einfluß der Herztätigkeit) des Patienten herrühren. Als Kriterium für ein Pulsschlagsignal wird die Zeit zwischen seinem Maximum und seinem Minimum und die Differenz des maximalen und minimalen Gasflusses herangezogen. Wird ein Pulsschlagsignal erkannt, so wird für eine kurze Zeit (bevorzugt 1,5 s) die Überwachung der monotonen Steigung des Atemgasflusses außer Betrieb gesetzt oder unempfindlicher eingestellt. Zusätzlich oder alternativ können bei Erkennen eines derartigen Pulsschlagsignals die Schwellenwerte für den Vergleich der 1. Ableitung des Atemgasflusses verändert, vorzugsweise unempfindlicher eingestellt werden.

Die Vorteile liegen sowohl in einer geringeren Störanfälligkeit als auch in einer höheren Genauigkeit beim Schalten in die Ein- oder Ausatmungsphase in der CPAP-Therapie.

Im folgenden wird die Erfindung anhand der Zeichnungen näher erläutert. Es zeigen:

Fig. 1    Diagramme zur Erläuterung des Verfahrens beim Schalten von der Ausatmungsphase in die Einatmungsphase,

Fig. 2    Diagramme zur Erläuterung des Verfahrens beim Schalten von der Einatmungsphase in die Ausatmungsphase, und

Fig. 3-5    weitere Ausführungsformen des Verfahrens.

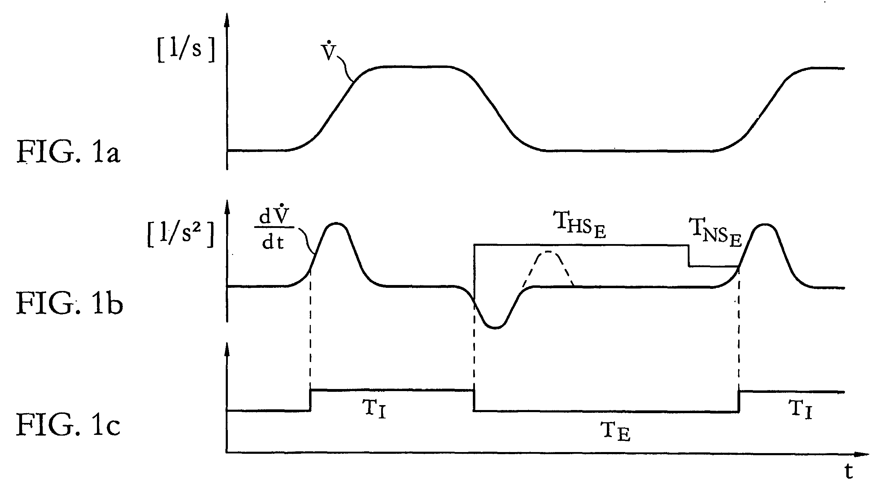

Das Diagramm Fig. 1a) zeigt den zeitlichen Verlauf der Atemgasflußkurve $\dot{V}$ (z.B. in 1/s). Fig. 1b) zeigt die erste Ableitung der Atemgasflußkurve nach der Zeit $d\dot{V}/dt$; und Fig. 1c) zeigt einen vergrößerten Ausschnitt der aus der ersten Ableitung abgeleitete Folge von Einatmungsphase (Inspiration) und Ausatmungsphase (Expiration) $T_I$ bzw $T_E$.

In Fig. 1b) wird der Übergang von der Aus- in die Einatmungsphase bezüglich zweier Schwellenwerte $HS_E$ und $NS_E$ dargestellt. Anschließend an die Schaltung in die Ausatmungsphase $T_E$ wird ein hoher Schwellenwert $HS_E$ für das Auslösen der nächsten Schaltung in die Einatmungsphase festgesetzt. Es ist zu erkennen, daß ein eventuelles Überschwingen (gestrichelte Linie) den Schwellenwert $HS_E$ nicht erreicht und somit keine irrtümliche Schaltung auslösen kann. Nach einer von der Zeitdauer der vorangehenden Inspiration abhängigen Zeitdauer $T_{HS(E)}$, die vorzugsweise das 0,9-fache bis 1,5-fache, bevorzugt das 1,0-fache der Dauer der vorhergehenden Einatmungsphase $T_I$ ist, wird ein niedriger Schwellenwert $NS_E$ angesetzt. In diesem Bereich, wo der Übergang in die andere Atmungsphase stattfindet, gewährleistet der niedrige, empfindlichere Schwellenwert eine genaue Bestimmung des Zeitpunkts des Übergangs. Um eine zu lange andauernde Insensibilität eines entsprechenden Geräts zu vermeiden, wird die Dauer der hohen Schwelle $T_{HS(E)}$ in der Ausatmungsphase $T_E$ auf ca. 3 Sekunden begrenzt. Außerdem kann zu Beginn der Ausatmungsphase für eine Zeitdauer von etwa 1 Sekunde die Einatmung verhindert werden.

Das Festsetzen des niedrigen Schwellenwertes NSE in der Ausatmungsphase erfolgt nur bei einem positiven Wert des Gasflusses bezüglich einer vorherigen Kalibrierung des Systems auf den Gasfluß "Null". Ist das nicht der Fall, so wird das Inkrafttreten des Schwellenwertes NS um jeweils 100 ms verzögert, bis ein positiver Wert festgestellt wird.

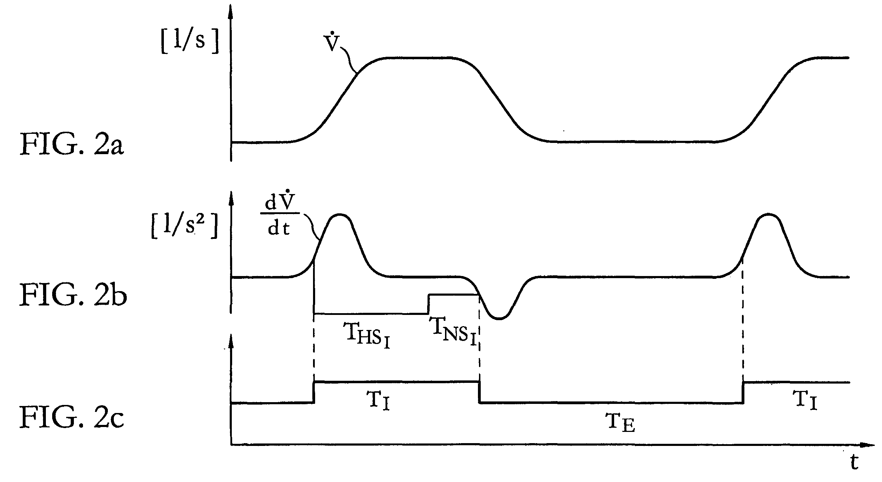

In Fig. 2a), ist der zeitliche Verlauf der Atemgasflußkurve V dargestellt; Fig. 2b) zeigt die erste Ableitung der Atemgasflußkurve, und Fig. 2c) zeigt die aus der ersten Ableitung gewonnene Folge der Ein- und Ausatmungsphase.

In Fig. 2 wird der Übergang von der Ein- in die Ausatmungsphase bezüglich der Schwellenwerte $HS_I$ und $NS_I$ dargestellt. Der hohe Schwellenwert $HS_I$ bedeutet das Zulassen einer Abweichung von der ersten Ableitung zu niedrigeren Werten als beim zweiten Schwellenwert $NS_I$. Nach dem Beginn der Einatmungsphase ist also die Schaltung unempfindlicher gegen Abweichungen in der Intensität der ersten Ableitung zu geringeren Werten, die ein unerwünschtes Schalten in die Ausatmungsphase einleiten könnten. Wie in Fig. 1 bewirkt die geringere Abweichung des niedrigen Schwellenwertes von der 1. Ableitung eine erhöhte Empfindlichkeit gegenüber dem Abfallen der Kurve der ersten Ableitung, die den Übergang in die Ausatmungsphase anzeigt.

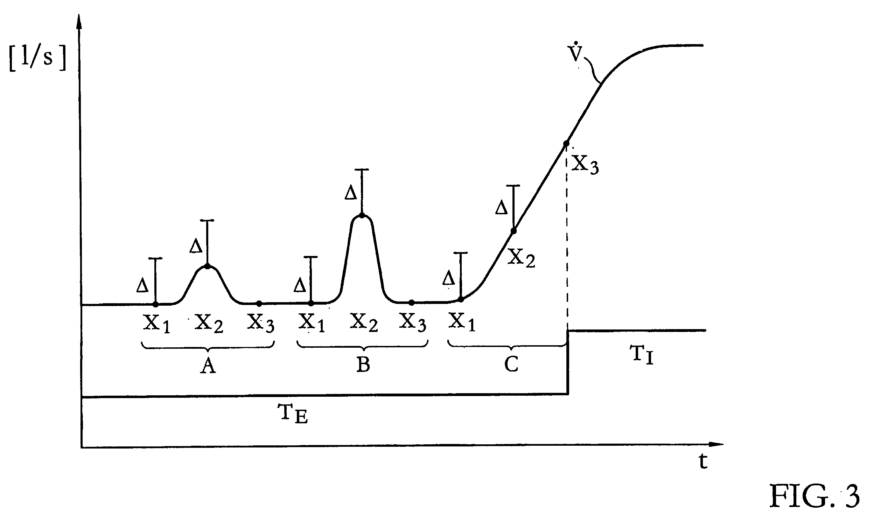

Fig. 3 zeigt eine Ausführungsform, bei der zusätzlich zu dem in Fig. 1 dargestellten Verfahren in der Ausatmungsphase während des Vergleichens der ersten Ableitung mit dem zweiten niedrigen Schwellenwert $NS_E$ die Gasflußkurve $\dot{V}$ in einer Folge von mindestens drei Abtastwerten $X_1$, $X_2$ und $X_3$ abgetastet wird, um eine monotone Steigung des Atemgasflusses zu ermitteln. Für das Schalten in die Einatmungsphase $T_I$ ist dabei die Bedingung gesetzt, daß bei jeweils drei Abtastwerten $X_1$ bis $X_3$ der zweite und der dritte Abtastwert $X_2$ bzw. $X_3$ den jeweils vorangegangenen Abtastwert $X_1$ bzw. $X_2$ jeweils vergrößert um einen festgelegten Betrag $\Delta$ überschreiten müssen; d.h. es müssen die beiden Bedingungen $X_1 + \Delta < X_2$ und $X_2 + \Delta < X_3$ erfüllt sein, wobei $\Delta$ ein geeignet voreingestellter Wert ist. Aus Fig. 3 ist zu erkennen, daß lediglich die letzte Folge C von $X_1$ bis $X_3$-Werten, die auf der Flanke des Einatmungsgasflusses liegt, diese Bedingung erfüllt. Bei der ersten Folge A von $X_1$ bis $X_3$-Werten erfüllt bereits der $X_2$-Wert das genannte Kriterium nicht, und bei der zweiten Folge B von $X_1$ bis $X_3$-Werten wird zwar das Kriterium im Wert $X_2$, jedoch von $X_3$ nicht erfüllt. Auf diese Weise lassen sich kurzzeitige Schwankungen, die z.B. durch den Pulsschlag des Patienten hervorgerufen werden können, und in der ersten Ableitung der Gasflußkurve V relativ groß erscheinen, erkennen, so daß das Schalten in die Einatmungsphase durch diese fehlerhaften Signale nicht ausgelöst wird. Die Abtastwerte sollen einen zeitlichen Abstand aufweisen, der größer als die Anstiegsdauer einer Pulswelle des Patienten ist; vorzugsweise beträgt der zeitliche Abstand der Abtastwerte 200 bis 300 ms.

Durch eine zeitliche Begrenzung der Einatmungsphase von z.B. 3 bis 4 s wird danach unabhängig von der Schaltauslösung durch das Gasflußsignal oder durch die Ableitung des Gasflußsignals in jedem Fall in die Ausatmungsphase umgeschaltet. Dieses Verfahren führt zum Schalten in die Einatmungsphase auch z.B. in solchen Fällen, wenn eine so flache Atmung vorhanden ist, daß die Werte der Gasflußkurve in der Einatmungsphase den niedrigen Schwellenwert $NS_I$ nicht erreichen.

Durch eine zeitliche Begrenzung der Ausatmungsphase von z.B. 1 bis 12 s wird danach unabhängig von der Schaltauslösung durch das Gasflußsignal oder durch die Ableitung des Gasflußsignals in jedem Fall in die Einatmungsphase umgeschaltet. Dieses Verfahren führt zum Schalten in die Einatmungsphase auch z.B. in solchen Fällen, auch wenn keine Spontanatmung des Patienten vorhanden ist oder nicht erkannt wird, so daß die Werte der Gasflußkurve in der Einatmungsphase den niedrigen Schwellenwert $NS_E$ nicht erreichen.

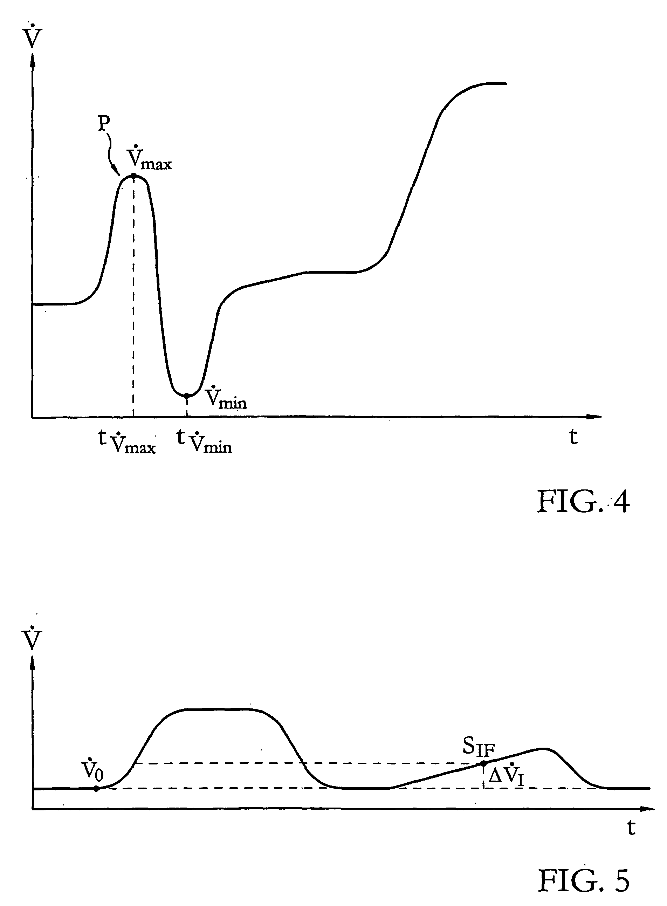

Fig. 4 zeigt eine weitere Ausführungsform, wobei unerwünschte Pulsschlagsignale, die von der Herztätigkeit des Patienten herrühren, in der Atemgasflußkurve $V(t)$ erkannt werden können. Der Pulsschlag wirkt sich in Form einer Schwingung mit steilen Flanken und entsprechender Amplitude aus, die der Gasflußkurve $V$ überlagert ist. Wenn die Steilheit der Flanke und damit die 1. Ableitung dieses Signals den Schwellenwert für das Umschalten in die Einatmungsphase erreicht, kann vorzeitig ein unerwünschtes Umschalten in die Einatmungsphase erfolgen. Um ein solches fehlerhaftes Umschalten zu vermeiden, wird mit Hilfe des Verfahrens ein Pulsschlagsignal P erkannt, wenn die folgenden Bedingungen erfüllt sind:

$$\dot{V}_{max} - \dot{V}_{min} \geq \Delta \dot{V}_P \tag{1}$$

$$t_{\dot{V}min} - t_{\dot{V}max} \leq \Delta t_p \tag{2}$$
$$\text{mit } \Delta t_p > 0$$

$\dot{V}_{max} =$ lokales Maximum das Gasflusses,
$\dot{V}_{min} =$ lokales Minimum des Gasflusses,
$t_{\dot{V}max} =$ Zeitpunkt des maximalen Gasflusses,
$t_{\dot{V}min} =$ Zeitpunkt des minimalen Gasflusses,
$\Delta V_P =$ empirisch bestimmter Schwellenwert für die Änderung des Gasflusses infolge eines Pulsschlagsignals.
$\Delta t_p =$ maximaler zeitlicher Abtand der aufeinanderfolgenden Maxima und Minima des Pulsschlagsignals

$\Delta \dot{V}_p$ beträgt vorzugsweise 0,5 bis 5 % des maximalen Gasflusses in der Einatmungsphase, $\Delta t_p$ beträgt vorzugsweise 400 bis 600 ms, besonders bevorzugt 500 ms.

Wenn die Bedingungen (1) und (2) erfüllt sind, so wird für 1 bis 3 Sekunden, vorzugsweise für 1,5 Sekunden die Überwachung der monotonen Steigung des Atemgasflusses außer Betrieb gesetzt oder unempfindlicher eingestellt, so daß ein irrtümliches Schalten verhindert wird. Zusätzlich oder alternativ können bei Erkennen eines derartigen Pulsschlagsignals die Schwellenwerte für den Vergleich der 1. Ableitung des Atemgasflusses verändert, vorzugsweise unempfindlicher eingestellt werden.

Bei Anwendung des Verfahrens nach Fig. 3 in Verbindung mit dem Verfahren nach Fig. 4 wird bei Erfüllung der Bedingungen (1) und (2) das Verfahren nach Fig. 3 für einen bestimmten Zeitraum, vorzugsweise für 1 bis 3 s, besonders bevorzugt für 1,5 s, ausgesetzt.

Fig. 5 zeigt eine weitere Ausführungsform, wobei ein Kriterium für das Umschalten in die Einatmungsphase bei flacher Atmung dargestellt ist. Die Atemgasflußkurve $\dot{V}(t)$ zeigt im Falle der Flachatmung einen langsamen Anstieg des Gasflusses in der Einatmungsphase. Die Flachatmung führt zu einer geringen ersten Ableitung, wodurch der Schwellenwert für das Umschalten in die Einatmungsphase nicht erreicht wird. Es wird daher für die Bestimmung des Schaltvorganges die Atemgasflußkurve $V$ direkt herangezogen, und es erfolgt ein Umschalten von der Ausatmungs- in die Einatmungsphase, wenn der Atemgasfluß $V$ einen vorbestimmten Schwellenwert $S_{IF}$ erreicht hat. Dabei gilt:

$$S_{IF} = \dot{V}_0 + \Delta \dot{V}_I \tag{3}$$

$\dot{V}_0 =$ Atemgasfluß beim letzten Umschalten in die Einatmungsphase vor dem Beginn der Flachatmung, insbesondere beim Umschalten durch Auswerten der 1. Ableitung des Atemgasflusses oder Überwachung der monotonen Steigung. In diesem Falle wird für die Ermittlung des Atemgasflusses $\dot{V}_0$ der Atemgasfluß beim

letzten Umschalten durch die 1. Ableitung herangezogen.

$\Delta \dot{V}_I$ =     empirisch bestimmter zusätzlicher Atemgasfluß in der Einatmungsphase, vorzugsweise 1 % - 60 %, besonders bevorzugt 2 bis 5 %, des maximalen Gasflusses in der Einatmungsphase bei normaler Atmung.

$\dot{V}_0$ wird vor der letzten Einatmungsphase gemessen und gespeichert.

Die in den Figuren 4 und 5 dargestellten Ausführungsformen können vorzugsweise in Verbindung mit den in den Figuren 1 bis 3 dargestellten Verfahren angewandt werden.

Bei der Verwendung des Verfahrens gemäß Fig. 5 bei einem Verfahren mit differentieller Auswertung der Atemgasflußkurve für das Bestimmen der Ausatmungs- und Einatmungsphase, wird sowohl die 1. Ableitung des Atemgasflusses als auch der Atemgasfluß selbst mit zugehörigen Schwellenwerten überwacht und in die Einatmungsphase umgeschaltet, wenn einer der beiden Parameter den zugehörigen Schwellenwert erreicht. Das Erreichen des Schwellenwertes $S_{IF}$ der Atemgasflußkurve ist dann das Kriterium für das Umschalten in die Einatmungsphase.

Die Erfindung betrifft eine Vorrichtung für die Anwendung des Verfahrens.

**Patentansprüche**

1.    Vorrichtung zum Erkennen von Pulsschlagsignalen (P) in der CPAP-Therapie, wobei ein Signal der Gasflußkurve $\dot{V}(t)$ dann als Pulsschlagsignal (P) erkannt wird, wenn die folgenden Bedingungen erfüllt sind:

$$(a)\ \dot{V}_{max} - \dot{V}_{min} \geq \Delta \dot{V}_P,$$

und

$$(b)\ t_{\dot{V}min} - t_{\dot{V}max} \leq \Delta t_P,$$

wobei $\Delta t_p > 0$ ist

$\dot{V}_{max}$ =     lokales Maximum des Gasflusses
$\dot{V}_{min}$ =     lokales Minimum des Gasflusses
$t_{\dot{V}max}$ =     Zeitpunkt des maximalen Gasflusses,
$t_{\dot{V}min}$ =     Zeitpunkt des minimalen Gasflusses
$\Delta V_P$ =     empirisch bestimmter Schwellenwert für die Änderung des Gasflusses infolge eines Pulsschlagsignals, vorzugsweise 5 bis 30 % des maximalen Gasflusses in der Einatmungsphase;
$\Delta t_p$ =     maximaler zeitlicher Abstand der aufeinanderfolgenden Maxima und Minima des Pulsschlagsignals, vorzugsweise 400 bis 600 ms.

2.    Vorrichtung nach Anspruch 1, wobei Mittel vorgesehen sind, um nach dem Erkennen des Pulsschlagsignals (P) die Überwachung einer monotonen Steigung des Atemgasflusses für ein vorbestimmtes Zeitintervall außer Betrieb zu setzen oder unempfindlicher einzustellen.

3.    Vorrichtung nach Anspruch 2, wobei das vorbestimmte Zeitintervall 1 bis 3 s, vorzugsweise 1,5 s, beträgt.

4.    Vorrichtung nach Anspruch 1, 2 oder 3 in Kombination mit einer Vorrichtung zum Schalten in die Ein- oder Ausatmungsphase ($T_I$ bzw. TE) in der CPAP-Therapie, wobei Mittel vorgesehen sind, den Atemgasfluß ($\dot{V}$) vom und zum Patienten nach der Zeit zur differenzieren, wobei
nach dem Umschalten in die jeweilige Atmungsphase die erste Ableitung ($d\dot{V}/dt$) des Glasflusses ($\dot{V}$) zuerst mit einem ersten hohen Schwellenwert ($HS_e$, $HS_I$) und anschließend mit einem zweiten niedrigen Schwellenwert ($NS_E$, $NS_I$) für eine Variation der ersten Ableitung verglichen wird, wobei das Schalten in die jeweilige andere Atmungsphase dann erfolgt, wenn die erste Ableitung den zweiten Schwellenwert ($NS_E$, $NS_I$) erreicht hat.

5.    Vorrichtung nach Anspruch 4, wobei Mittel vorgesehen sind, zusatzlich zum Vergleichen der ersten Ableitung mit dem zweiten niedrigen Schwellenwert ($NS_E$) in der Ausatmungsphase den zeitlichen Verlauf des Gasflusses ($\dot{V}$) in einer Folge von mindestens drei Abtastwerten ($X_1$, $X_2$, $X_3$) abzutasten, wobei, wenn die beiden Bedingungen

$$X_1 + \Delta < X_2 \text{ und } X_2 + \Delta < X_3$$

$\Delta$ = vorgegebener Zuwachs erfüllt sind, beim Wert $X_3$ das Schalten in die Einatmungsphase erfolgt, wenn nicht vorher bereits das Schalten durch Vergleichen der ersten Ableitung mit dem niedrigen Schwellenwert ($NS_E$) erfolgte.

**6.** Vorrichtung nach Anspruch 5, wobei die Abtastwerte ($X_1$, $X_2$ und $X_3$) einen zeitlichen Abtand aufweisen, der größer als die Anstiegsdauer einer Pulswelle des Patienten ist.

**7.** Vorrichtung nach Anspruch 5 oder 6, wobei der zeitliche Abstand der Abtastwerte ($X_1$, $X_2$ und $X_3$) bevorzugt 200 bis 300 ms beträgt.

**8.** Vorrichtung nach einem der Ansprüche 4 bis 7, wobei die Zeitdauer ($T_{HS}$) des Vergleichens der ersten Ableitung mit dem hohen Schwellenwert ($HS_E$, $NS_T$) größer als die Zeitdauer ($T_{NS}$) des Vergleichens mit dem niedrigen Schwellenwert ($NS_E$, $NS_I$) ist.

**9.** Vorrichtung nach Anspruch 8, wobei in der Ausatmungsphase gilt:

$$T_I \leq T_{HS(E)} < 4 \text{ s.}$$

**10.** Vorrichtung nach Anspruch 9, wobei bevorzugt

$$T_{HS(E)} \geq (0{,}9 \text{ bis } 1{,}5) \times T_I \,,$$

vorzugsweise

$$T_{HS(E)} \geq 1{,}0 \, T_I$$

ist.

**11.** Vorrichtung nach einem der Ansprüche 4 bis 10, wobei in der Einatmungsphase gilt:

$$0{,}5 \text{ s} \leq T_{HS(I)} < 3 \text{ s.}$$

**12.** Vorrichtung nach einem der Ansprüche 4 bis 11, wobei Mittel vorgesehen sind, um zu Beginn der Ausatmungsphase eine Einatmung für 1 Sekunde lang zu verhinder.

**13.** Vorrichtung nach einem der Ansprüche 4 bis 12, wobei Mittel vorgesehen sind, um das Festsetzen des niedrigen Schwellenwertes ($NS_E$) in der Ausatmungsphase nur bei einem positiven Wert des Gasflusses ($\dot{V}$) durchzuführen und im gegenteiligen Fall das Einsetzen des Sehwellenwertes ($NS_E$) um jeweils 100 ms zu verzögern, bis ein positiver Wert festgestellt wird.

**14.** Vorrichtung nach einem der Ansprüche 4 bis 13, wobei Mittel vorgesehen sind, um wenn vorner kein Umschalten in die Ausatmungsphase erfolgt, die Einatmungsphase vorzugsweise nach 4 s abzubrechen und in die Ausatmungsphase umzuschalten.

**15.** Vorrichtung nach einem der Ansprüche 4 bis 14, wobei Mittel vorgesehen sind, um wenn vorner kein Umschalten in die Einatmungsphase erfolgt, die Ausatmungsphase vorzugsweise nach 1 bis 12 s abzubrechen und in die Einatmungsphase umzuschalten.

**16.** Vorrichtung nach einem der Ansprüche 14 bis 15 wobei Mittel vorgesehen sind, um ein Umschalten von der Ausatmungsphase in die Einatmungsphase bei flacher Atmung dann durchzuführen, wenn der Atemgasfluß $\dot{V}$ einen vorbestimmten Schwellenwert $S_{IF}$ erreicht.

**17.** Vorrichtung nach Anspruch 16, wobei gilt:

$$S_{IF} = \dot{V}_0 + \Delta \dot{V}_I$$

$\dot{V}_0 =$      Atemgasfluß beim letzten Umschalten in die Einatmungsphase vor dem Beginn der Flachatmung, insbesondere beim Umschalten durch Auswerten der 1. Ableitung des Atemgasflusses. In diesem Falle wird für die Ermittlung des Atemgasflusses $\dot{V}_0$ der Atemgasfluß beim letzten Umschalten durch die 1. Ableitung herangezogen.

$\Delta \dot{V}_I =$      empirisch bestimmter zusätzlicher Atemgasfluß in der Einatmungsphase, vorzugsweise 30 % - 60 %, besonders bevorzugt 50 %, des maximalen Gasflusses in der Einatmungsphase bei normaler Atmung.

**Claims**

**1.** Apparatus for detecting pulse beat signals (P) in CPAP therapy wherein a signal of the gas flow curve V (t) is detected as a pulse beat signal (P) when the following conditions are fulfilled:

$$(a) \; \dot{V}_{max} - \dot{V}_{min} \geq \Delta \dot{V}_p,$$

and

$$(b) \; t_{\dot{V}min} - t_{\dot{V}max} \leq \Delta t_p,$$

wherein $\Delta t_p > 0$

$\dot{V}_{max}$ = local maximum of the gas flow
$\dot{V}_{min}$ = local minimum of the gas flow
$t_{\dot{V}max}$ = time of the maximum gas flow
$t_{\dot{V}min}$ = time of the minimum gas flow
$\Delta V_p$ = empirically determined threshold value for the change in the gas flow as a consequence of a pulse beat signal, preferably 5 to 30% of the maximum gas flow in the inspiration phase,
$\Delta t_p$ = maximum time interval between the successive maxima and minima of the pulse beat signal, preferably 400 to 600 ms.

**2.** Apparatus according to claim 1 wherein there are provided means for stopping monitoring of a monotonic rise in the respiratory gas flow or making it less sensitive for a predetermined period of time, after detection of the pulse beat signal (P).

**3.** Apparatus according to claim 2 wherein the predetermined period of time is 1 to 3s, preferably 1.5s.

**4.** Apparatus according to claim 1, claim 2 or claim 3 in combination with an apparatus for switching into the inspiration or expiration phase ($T_I$ and $T_E$ respectively) in CPAP therapy, wherein there are provided means for differentiating the respiratory gas flow ($\dot{V}$) from and to the patient with respect to time, wherein
after switching over into the respective respiration phase the first derivative ($d\dot{V}/dt$) of the gas flow ($\dot{V}$) is firstly compared with a first high threshold value ($HS_E$, $HS_I$) and then a second low threshold value ($NS_E$, $NS_I$) for a variation in the first derivative, wherein switching into the respective other respiration phase takes place when the first derivative has reached the second threshold value ($NS_E$, $NS_I$).

**5.** Apparatus according to claim 4 wherein there are provided means for sampling, in addition to the comparison of the first derivative with the second low threshold value ($NS_E$) in the expiration phase, the variation in respect of time of the gas flow ($\dot{V}$) in a sequence of at least three sampling values ($X_1$, $X_2$, $X_3$), wherein if the two conditions

$$X_1 + \Delta < X_2 \text{ and } X_2 + \Delta < X_3$$

and

$\Delta$ = predetermined increase

are fulfilled switching into the inspiration phase occurs at the value $X_3$ unless switching was already previously effected by comparison of the first derivative with the low threshold value ($NS_E$).

6. Apparatus according to claim 5 wherein the sampling values ($X_1$, $X_2$, $X_3$) are at a spacing in respect of time which is greater than the rise time of a pulse wave of the patient.

7. Apparatus according to claim 5 or claim 6 wherein the spacing in respect of time of the sampling values ($X_1$, $X_2$ and $X_3$) is preferably 200 to 300 ms.

8. Apparatus according to one of claims 4 to 7 wherein the duration ($T_{HS}$) of the comparison of the first derivative with the high threshold value ($HS_E$, $HS_I$) is greater than the duration ($T_{NS}$) of the comparison with the low threshold value ($NS_E$, $NS_I$).

9. Apparatus according to claim 8 wherein the following applies in the expiration phase:

$$T_I \leq T_{HS(E)} < 4 \text{ s.}$$

10. Apparatus according to claim 9 wherein preferably

$$T_{HS(E)} \geq (0.9 \text{ to } 1.5) \times T_I,$$

preferably

$$T_{HS(E)} \geq 1.0 \, T_2.$$

11. Apparatus according to one of claims 4 to 10 wherein the following applies in the inspiration phase:

$$0.5 \text{ s} \leq T_{HS(I)} < 3 \text{ s.}$$

12. Apparatus according to one of claims 4 to 11 wherein there are provided means for preventing an inspiration for 1 second at the beginning of the expiration phase.

13. Apparatus according to one of claims 4 to 12 wherein there are provided means for fixing the low threshold value ($NS_E$) in the expiration phase only at a positive value in respect of the gas flow ($\dot{V}$) and in the opposite case for delaying setting of the threshold value ($NS_E$) by 100 ms in each case until a positive value is established.

14. Apparatus according to one of claims 4 to 13 wherein there are provided means for breaking off the inspiration phase preferably after 4 seconds and switching over into the expiration phase, if no switching-over into the expiration phase occurs previously.

15. Apparatus according to one of claims 4 to 14 wherein there are provided means for breaking off the expiration phase preferably after 1 to 12 seconds and switching over into the inspiration phase, if no switching-over into the inspiration phase occurs previously.

16. Apparatus according to one of claims 4 to 15 wherein there are provided means for effecting a switch from the expiration phase into the inspiration phase with shallow respiration when the respiratory gas flow V reaches a predetermined threshold value $S_{IF}$.

17. Apparatus according to claim 16 wherein the following applies:

$$S_{IF} = \dot{V}_0 + \Delta\dot{V}_I$$

$\dot{V}_0$ = respiratory gas flow at the last switch over into the inspiration phase prior to the beginning of shallow respiration, in particular at the switch over by evaluation of the first derivative of the respiratory gas flow, in that case the respiratory gas flow at the last switch over by the first derivative is used for determining the respiratory gas flow $\dot{V}_0$, and

$\Delta\dot{V}_I$ = an empirically determined additional respiratory gas flow in the inspiration phase, preferably 30% - 60%, particularly preferably 50%, of the maximum gas flow in the inspiration phase with normal respiration.

**Revendications**

1. Dispositif pour reconnaître des signaux de battement de pouls dans le traitement par ventilation spontanée en pression positive continue (thérapie CPAP), un signal de la courbe du flux de gaz $\dot{V}(t)$ étant alors reconnu comme signal de battement de pouls (P), lorsque les conditions suivantes sont réunies :

$$\text{(a)} \ \dot{V}_{max} - \dot{V}_{min} \geq \Delta\dot{V}_P,$$

et

$$\text{(b)} \ t\dot{V}_{min} - t\dot{V}_{max} \leq \Delta t_P,$$

$\Delta t_P$ étant > 0

$\dot{V}_{max}$ = maximum local du flux de gaz
$\dot{V}_{min}$ = minimum local du flux de gaz
$t\dot{V}_{max}$ = instant du flux de gaz maximal
$t\dot{V}_{min}$ = instant du flux de gaz minimal

$\Delta\dot{V}_P$ = valeur de seuil déterminée empiriquement pour la modification du flux de gaz à la suite d'un signal de battement de pouls, de préférence 5 à 30 % du flux de gaz maximum dans la phase inspiratoire ;

$\Delta t_P$ = écart maximal dans le temps des maxima et minima du signal de battement de pouls se succédant, de préférence 400 à 600 ms.

2. Dispositif selon la revendication 1, des moyens étant prévus pour mettre hors service ou rendre moins sensible la surveillance d'une augmentation monotone du flux de gaz respiratoire pendant un intervalle de temps prédéterminé après la reconnaissance du signal de battement de pouls (P).

3. Dispositif selon la revendication 2, l'intervalle de temps prédéterminé étant de 1 à 3 s, de préférence 1,5 s.

4. Dispositif selon l'une quelconque des revendications 1, 2 ou 3 en combinaison avec un dispositif pour passer en phase inspiratoire ou expiratoire ($T_I$ ou TE) dans la thérapie CPAP, des moyens étant prévus pour différencier dans le temps le flux de gaz respiratoire ($\dot{V}$) venant du et allant vers le patient, après le passage dans la phase respiratoire donnée, la première dérivation ($d\dot{V}/dt$) du flux de gaz ($\dot{V}$) étant d'abord comparée à une première valeur de seuil élevée ($HS_E$, $HS_I$) et ensuite à une deuxième valeur de seuil basse ($NS_E$, $NS_I$) pour une variation de la première dérivation, le passage dans l'autre phase respiratoire donnée se faisant lorsque la première dérivation a atteint la seconde valeur de seuil ($NS_E$, $NS_I$).

5. Dispositif selon la revendication 4, des moyens étant prévus en plus de la comparaison de la première dérivation avec la seconde valeur de seuil basse ($NS_E$) pour palper dans la phase expiratoire l'évolution dans le temps du flux de gaz ($\dot{V}$) dans une succession d'au moins trois valeurs de palpage ($X_1$, $X_2$, $X_3$) et si les deux conditions

$$X_1 + \Delta < X_2 \text{ et } X_2 + \Delta < X_3$$

$\Delta$ = accroissement prédéterminé

sont réunies, le passage dans la phase respiratoire se fait à la valeur $X_3$ à condition que le passage n'ait pas déjà eu lieu avant par la comparaison de la première dérivation avec la valeur de seuil basse ($NS_E$).

**6.** Dispositif selon la revendication 5, les valeurs de palpage ($X_1$, $X_2$ et $X_3$) présentant un écart dans le temps qui est plus grand que la durée d'augmentation d'une onde de pouls du patient.

**7.** Dispositif selon les revendications 5 ou 6, l'écart dans le temps des valeurs de palpage ($X_1$, $X_2$ et $X_3$) étant de préférence 200 à 300 ms.

**8.** Dispositif selon l'une quelconque des revendications 4 à 7, la durée dans le temps ($T_{HS}$) de la comparaison de la première dérivation avec la valeur de seuil élevée ($HS_E$, $NS_I$) étant plus longue que la durée dans le temps ($T_{NS}$) avec la valeur de seuil basse ($NS_E$).

**9.** Dispositif selon la revendication 8, la formule dans la phase expiratoire étant :

$$T_I \leq T_{HS(E)} < 4 \text{ s.}$$

**10.** Dispositif selon la revendication 9, la formule préférée étant

$$T_{HS(E)} \geq (0{,}9 \text{ à } 1{,}5) \times T_I,$$

de préférence

$$T_{HS(E)} \geq 1{,}0 \, T_I.$$

**11.** Dispositif selon l'une quelconque des revendications 4 à 10, la formule dans la phase inspiratoire étant :

$$0{,}5 \text{ s} \leq T_{HS(I)} < 3 \text{ s.}$$

**12.** Dispositif selon l'une quelconque des revendications 4 à 11, des moyens étant prévus pour empêcher pendant une seconde une inspiration au début de la phase expiratoire.

**13.** Dispositif selon l'une quelconque des revendications 4 à 12, des moyens étant prévus pour déterminer la valeur de seuil basse ($NS_E$) dans la phase expiratoire seulement lorsque la valeur du flux de gaz ($\dot{V}$) est positive et, dans le cas contraire, retarder l'intervention de la valeur de seuil ($NS_E$) de 100 ms jusqu'à ce qu'une valeur positive soit constatée.

**14.** Dispositif selon l'une quelconque des revendications 4 à 13, des moyens étant prévus pour que, si préalablement un passage dans la phase expiratoire n'a pas eu lieu, la phase inspiratoire est interrompue de préférence après 4 s, et pour passer à la phase expiratoire.

**15.** Dispositif selon l'une quelconque des revendications 4 à 14, des moyens étant prévus pour interrompre la phase expiratoire de préférence après 1 à 12 s à condition qu'un passage dans la phase inspiratoire n'ait pas déjà eu lieu avant et passer dans la phase inspiratoire.

**16.** Dispositif selon l'une quelconque des revendications 4 à 15, des moyens étant prévus pour passer de la phase expiratoire dans la phase inspiratoire lorsque la respiration est plate à condition que le flux de gaz respiratoire $\dot{V}$ atteigne une valeur de seuil $S_{IF}$ prédéterminée.

**17.** Dispositif selon la revendication 16, la formule étant :

$$S_{IF} = \dot{V}_0 + \Delta \dot{V}_1$$

$\dot{V}_0 =$ le flux de gaz lors du dernier passage dans la phase inspiratoire avant le début de la respiration plate, notamment lors du passage suite au dépouillement de la première dérivation du flux de gaz respiratoire. Dans ce cas la référence pour la détermination du flux de gaz respiratoire $\dot{V}_0$ est le flux de gaz respiratoire lors du dernier passage par la première dérivation.

$\Delta\dot{V}_I =$ le flux de gaz respiratoire supplémentaire empiriquement déterminé dans la phase inspiratoire, de préférence 30 % à 60 %, 50 % étant notamment préférés, du flux de gaz maximal dans la phase inspiratoire sous respiration normale.

FIG. 1a

FIG. 1b

FIG. 1c

FIG. 2a

FIG. 2b

FIG. 2c

FIG. 3

FIG. 4

FIG. 5